# EUROPÄISCHE PATENTSCHRIFT

(11) **EP 1 240 908 B1**
(45) Veröffentlichungstag und Bekanntmachung des Hinweises auf die Patenterteilung: **31.05.2006**
(21) Anmeldenummer: 01129733.0
(22) Anmeldetag: 13.09.1991
(51) Int. Cl.: A61L 24/00

(54) **Mehrkomponentenmaterial und Verfahren zu seiner Herstellung**
Multiple component material and process for producing the same
Matériau a composants multiples et son procédé de production

(30) Priorität: 13.09.1990 DE 4029136; 19.09.1990 DE 4029714
(43) Veröffentlichungstag der Anmeldung: 18.09.2002
(62) Teilanmeldung aus: 91916472.3
(73) Patentinhaber: DRAENERT, Klaus, Dr.med., D-81545 München (DE)
(72) Erfinder: DRAENERT, Klaus, Dr.med., D-81545 München (DE)

(56) Entgegenhaltungen:
- EP-A- 0 047 971
- EP-A- 0 050 215

## Beschreibung

Die Erfindung betrifft ein Mehrkomponentenmaterial , insbesondere ein Granulatanteil enthaltendes Implantationsmaterial , ein Verfahren zur Herstellung des Materials und die Verwendung des Implantationsmaterials zur Verankerung von Prothesenkomponenten im Knochen , zur Versteifung von Knochen , als Dübel von Knochenschrauben und als Implantat zur Verankerung von Schrauben. Die Polymerkomponente des erfindungsgemässen Implantationsmaterials ist vorzugsweise auf Acrylat-oder Polymethacrylatbasis oder einem Copolymer eines Acrylates und eines Methacrylates oder einer Mischung daraus aufgebaut. Die Polymer- bzw. Copolymerkomponente besteht dabei aus 1-50% aus unregelmässig geformten Granulatteilchen ohne eingeschlossene Füllerpartikel .

### Hintergrund der Erfindung

Die vorbestehenden Implantationsmaterialien sind als Knochenzemente bekannt und klinisch erprobt. Sie werden vom Körper nicht resorbiert , sind jedoch biologisch inert und werden in das Knochengerüst integriert. Bekannte Knochenzemente bestehen aus einer polymeren Komponente , die in der Regel in Form von Kügelchen vorliegt und auch als Perlpolymerisat bezeichnet wird , einer monomeren Komponente und gegebenenfalls einem Polymerisationskatalysator , einem Stabilisator und einem Beschleuniger. Vor dem Gebrauch werden die Komponenten möglichst homogen vermischt. Knochenzemente auf Acrylatbasis haben sich als vorteilhafte Verankerungswerkstoffe für Prothesenkomponenten erwiesen. Der Nachteil derartiger Knochenzemente kann jedoch darin bestehen , dass trotz verbesserter Misch- und Applikationsmethoden die Materialfestigkeit und die Reproduzierbarkeit noch nicht verlässlich optimiert werden konnten. Die Knochenzemente können im klinischen Verlauf ermüden und zeigen eine zunehmende Zerrüttung , in deren Folge Knochenresorption und die dann nicht zu vermeidende Auslockerung des Implantates auftreten können.

Einer solchen Polymerkomponente sind Füllerpartikel beigemischt, beispielsweise Röntgenkontrastmittel oder auch Antibiotika. In einer Normalpackung Knochenzement mit 40g Polymerpulver sind beispielsweise bis zu 6g Röntgenkontrastmittel , üblicherweise in Form von Bariumsulfat oder Zirkondioxid zugesetzt. Aus der DE-A-2905878 ist bekannt, dass auch Hydroxylapatit in entsprechender Beimengung einen ausreichenden Röntgenkontrast des Knochenzementimplantates ergeben kann. Lee A.J.C., Ling R.S.M. and Vangala S.S. haben nachgewiesen , dass Röntgenkontrastmittel und Antibiotika zu einer deutlichen Schwächung der Materialfestigkeit des Knochenzementes führen , welche zwischen 4% und 25% liegen kann (Arch.Orthop.Traumat.Surg.92, 1-18, 1978).

Es liegt dieser Erfindung die Aufgabe zugrunde , ein Verfahren zur Herstellung von partikelförmigen Zusätzen und ein Implantationsmaterial mit partikelförmigen Zusätzen bereitzustellen.

Diese Aufgabe wird durch das Mehrkomponentenmaterial , insbesondere das Implantationsmaterial , und das Verfahren gemäss der Erfindung gelöst.

In der vorliegenden Beschreibung wird der Begriff Polymer so verstanden , dass dabei auch Copolymere umfasst werden. Vorzugsweise werden erfindungsgemäss Polymere auf der Basis eines Polyacrylats oder Polymethacrylats , Copolymere eines Acrylats und eines Methacrylates, Mischungen der genannten Polymere und Copolymere , Epoxydharze oder ein Polymer eines als Implantationsmaterial verwendbaren Kunststoffes verwendet.

Es hat sich gezeigt, dass beim Einsatz einer solchen Polymerkomponente Knochenzemente mit deutlich verbesserten mechanischen Eigenschaften erhalten werden. Es sollten zumindest 1-50 Gew.-% aus einem solchen Granulat bestehen , wobei mit 5 bis 20 Gew.-% schon sehr gute Resultate erzielt werden.

Ein solches Polymermaterial kann durch Zerkleinern aus einem kompakten Block hergestellt werden , beispielsweise als Granulat in geeigneter Teilchengrösse , aber auch durch Schmelzen von normalem Perlpolymerisat und Auspressen unter hohen Drücken , beispielsweise 300 bis 2000 bar, durch eine Düse ; die Temperaturen , die hierfür notwendig sind , müssen allerdings unter 255° Celsius liegen , damit das Polymer nicht verbrennt.

Dieses Herstellungsverfahren ist preisgünstig und bietet darüber hinaus auch den Vorteil , dass Ausgangsmaterialien , die bereits klinisch erprobt sind , für die Herstellung des Implantationsmaterials verwendet werden können.

## Patentansprüche

1. Implantationsmaterial zur Verwendung als Knochenzement bestehend aus einem Polymer und/oder Copolymer sowie Füllerpartikeln und einem Monomeranteil, gegebenenfalls mit einem Stabilisator , Katalysator und Beschleuniger , **dadurch gekennzeichnet, dass** Polymer bzw. Copolymer zumindest teilweise in Form von unregelmässig geformeten Granulatteilchen ohne eingeschlossene Füllerpartikel vorliegen.

2. Implantationsmaterial nach Anspruch 1 , **dadurch gekennzeichnet, dass** 1-50% der Polymerkomponente in Form von unregelmässig geformten Granulatteilchen ohne eingeschlossene Füllerpartikel vorliegt.

3. Implantationsmaterial nach Anspruch 1 und 2 , **dadurch gekennzeichnet , dass** die Polymerpartikel eine Grösse bis 300 µm aufweisen.

4. Implantationsmaterial nach Anspruch 1 , **dadurch gekennzeichnet , dass** das Granulat durch Zerkleinerung eines Polymerblockes gewonnen wurde.

5. Implantationsmaterial nach Anspruch 1, **dadurch gekennzeichnet , dass** das Granulat durch Zerkleinerung eines Polymerblockes , der Füllerpartikel enthält , gewonnen wurde.

6. Implantationsmaterial nach Anspruch 5 , **dadurch gekennzeichnet , dass** die Füllerpartikel in den Polymerblock in der Schmelze eingebracht wurden.

## Claims

1. An implantation material for use as a bone cement comprising a polymer and/or copolymer as well as filler particles and a monomer portion, if necessary with a stabilizer, catalyst and accelerator, **characterized in that** the polymer or copolymer as the case may be is at least partially present in the form of irregularly shaped granulate particles without embedded filler particles.

2. The implantation material according to claim 1, **characterized in that** 1-50 % of the polymer components is present in the form of irregularly shaped granulate particles without embedded filler particles.

3. The implantation material according to claim 1 and 2, **characterized in that** the polymer particles have a size up to 300 µm.

4. The implantation material according to claim 1, **characterized in that** the granulate is produced by comminution of a block of polymer.

5. The implantation material according to claim 1, **characterized in that** the granulate is produced by comminution of a block of polymer which contains filler particles.

6. The implantation material according to claim 5, **characterized in that** the filler particles in the block of polymer are put into the melt.

## Revendications

1. Matériau d'implantation destiné à l'utilisation en tant que ciment osseux se composant d'un polymère et/ou d'un copolymère ainsi que de particules de remplissage et d'une fraction de monomères, éventuellement avec un agent stabilisant, un catalyseur et un accélérateur, **caractérisé en ce que** le polymère, respectivement le copolymère, est présent au moins partiellement sous forme de particules de granulat de forme irrégulière sans particules incluses de remplissage.

2. Matériau d'implantation selon la revendication 1, **caractérisé en ce que** 1 à 50% des constituants polymérisés sont présents sous forme de particules de granulat de forme irrégulière sans particules incluses de remplissage.

3. Matériau d'implantation selon la revendication 1 ou 2, **caractérisé en ce que** les particules de polymère présentent une taille allant jusqu'à 300 µm.

4. Matériau d'implantation selon la revendication 1, **caractérisé en ce que** le granulat est obtenu par broyage d'un bloc de polymère.

5. Matériau d'implantation selon la revendication 1, **caractérisé en ce que** le granulat est obtenu par broyage d'un bloc de polymère qui renferme les particules de remplissage.

6. Matériau d'implantation selon la revendication 5, **caractérisé en ce que** les particules de remplissage renfermées dans le bloc de polymère sont incorporées dans le produit fondu.
